# EUROPEAN PATENT APPLICATION

(11) **EP 4 632 071 A1**
(43) Date of publication of application: **15.10.2025**
(21) Application number: 23912958.8
(22) Date of filing: 28.12.2023
(51) Int. Cl.: C12N 15/70, C12N 9/02, C12P 13/22

(54) **MICROORGANISM HAVING EXOGENOUS SOLUBLE PYRIDINE NUCLEOTIDE TRANSHYDROGENASE INTRODUCED THEREINTO AND METHOD FOR PRODUCING L-TRYPTOPHAN USING SAME**

(30) Priority: 28.12.2022 KR 20220187716
(71) Applicant: CJ CheilJedang Corporation, Seoul 04560 (KR)
(72) Inventor: PARK, Soe-hee, Seoul 04560 (KR); JUNG, Moo Young, Seoul 04560 (KR); PARK, Seul-Gi, Seoul 04560 (KR); KIM, Sang Jun, Seoul 04560 (KR); LEE, Sumin, Seoul 04560 (KR)
(74) Representative: Meissner Bolte Partnerschaft mbB
(86) International application number: PCT/KR2023/021783
(87) International publication number: WO 2024/144284

(57) **Abstract**

The present disclosure relates to a microorganism of the genus *Escherichia* into which an exogenous soluble pyridine nucleotide transhydrogenase is introduced, and a method for producing L-tryptophan using the same.

## Description

### [Technical Field]

The present disclosure relates to a microorganism of the genus *Escherichia,* into which an exogenous soluble pyridine nucleotide transhydrogenase is introduced, and a method for producing L-tryptophan using the same.

### [Background Art]

In order to produce L-amino acids and other beneficial substances, a variety of research has been performed to develop microorganisms with high-efficiency production and technologies for fermentation processes. For example, when producing L-tryptophan, target-specific approaches, such as a method of increasing expression of a gene encoding an enzyme involved in L-tryptophan biosynthesis or a method of removing a gene unnecessary for biosynthesis, have been mainly used (US 8945907 B2).

However, as the demand for L-tryptophan increases, research is still needed to effectively increase the L-tryptophan producing ability.

### [Disclosure]

### [Technical Problem]

The present inventors have confirmed that when an exogenous udhA protein is introduced into a microorganism, the L-tryptophan producing ability of the microorganism is increased compared to that of a non-modified microorganism, thereby completing the present disclosure.

### [Technical Solution]

It is one object of the present disclosure to provide a microorganism of the genus *Escherichia* into which an exogenous udhA protein or a polynucleotide encoding the protein is introduced.

It is another object of the present disclosure to provide a method for producing L-tryptophan, including: culturing a microorganism of the genus *Escherichia,* into which an exogenous udhA protein or a polynucleotide encoding the protein is introduced, in a medium.

It is still another object of the present disclosure to provide a composition for producing L-tryptophan, including a microorganism of the genus *Escherichia* into which an exogenous udhA protein or a polynucleotide encoding the protein is introduced; a medium on which the microorganism is cultured; or a combination thereof.

### [Advantageous Effects]

The microorganism of the present disclosure may have an increased L-tryptophan producing ability compared to an existing non-modified microorganism by introducing an exogenous udhA protein.

### [Detailed Description of Preferred Embodiments]

Hereinafter, the present disclosure will be described in detail. Meanwhile, each description and embodiment disclosed herein can be applied to other descriptions and embodiments with respect to common features. That is, all combinations of various elements disclosed herein fall within the scope of the present disclosure. Further, the scope of the present disclosure is not limited by the specific description described below. In addition, a number of papers and patent documents have been referenced and cited throughout the present specification. The content of the cited papers and patent documents is incorporated herein by reference in their entirety, and the level of technical field to which the present disclosure belongs and the contents of the present disclosure will be described more clearly.

One aspect of the present disclosure provides a microorganism of the genus *Escherichia* into which an exogenous udhA protein or a polynucleotide encoding the protein is introduced.

As used herein, the term "soluble pyridine nucleotide transhydrogenase (udhA)" refers to a protein having the activity of catalyzing the reaction of NAD⁺ + NADPH ↔ NADH + NADP⁺. The "soluble pyridine nucleotide trans hydrogenase" may be used interchangeably with "udhA protein", "udhA", *etc.*

The amino acid sequence of the udhA protein can be obtained from NCBI's GenBank, a known database, *etc.*

In one example, the udhA protein of the present disclosure may be derived from a microorganism. The protein may be derived from a microorganism selected from microorganisms of the genus *Pseudomonas* and the genus *Erwinia.* More specifically, it may be derived from a microorganism selected from *Pseudomonas aeruginosa* and *Erwinia amylovora.*

In another example, the amino acid sequence of the udhA protein of the present disclosure may be WP_003091177.1 derived from *Pseudomonas aeruginosa* and WP_004154881.1 derived from *Erwinia amylovora, etc.*

In the present disclosure, the udhA protein may have, include, or consist of an amino acid sequence of SEQ ID NO: 1 or 3, or may consist essentially of the amino acid sequence above.

In the present disclosure, the udhA protein may include the amino acid sequence of SEQ ID NO: 1 or 3, or an amino acid sequence having 70% or more, 75% or more, 76% or more, 85% or more, 86% or more, 87% or more, 87.5% or more, 88% or more, 89% or more, 90% or more, 91% or more, 92% or more, 92.5% or more, 93% or more, 94% or more, 95% or more, 96% or more, 97% or more, 97.5% or more, 98% or more, 99% or more, 99.5% or more, 99.7% or more, or 99.9% or more homology or identity thereto. In one example, the udhA protein of the present disclosure may include any one or more selected from the group consisting of any one amino acid sequence among SEQ ID NOS: 1, 3, and 5, or an amino acid sequence having 87.5% or more homology or identity thereto. Additionally, it is apparent that any protein having an amino acid sequence, in which part of the sequence is deleted, modified, substituted, conservatively substituted or added, may also fall within the scope of the present disclosure as long as the amino acid sequence has such a homology or identity, and exhibits an efficacy corresponding to the protein including the amino acid sequence of SEQ ID NO: 1 or 3.

For example, it may be sequence additions that do not alter the function of the protein of the present disclosure, naturally occurring mutations, silent mutations therein, or conservative substitutions at the N-terminus, C-terminus and/or within the amino acid sequences.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. Typically, conservative substitutions may have little or no effect on the activity of a protein or polypeptide.

As used herein, the term "homology" or "identity" refers to the degree of relatedness between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines the homology, similarity or identity as the value obtained by dividing the number of similarly aligned symbols (i.e., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

The udhA protein of the present disclosure may be encoded by *udhA* gene.

In one example, the *udhA* gene may be a polynucleotide encoding WP_003091177.1 derived from *Pseudomonas aeruginosa* or WP_004154881.1 derived from *Erwinia amylovora,* but is not limited thereto. In another example, the *udhA* gene may be NZ_CP034244.1 derived from *Pseudomonas aeruginosa* or NC_013961.1 derived from *Erwinia amylovora,* but is not limited thereto, and it is apparent that the *udhA* gene may include *udhA* genes of various origins encoding the protein having the soluble pyridine nucleotide transhydrogenase activity.

As used herein, the term "polynucleotide", which is a polymer of nucleotides composed of nucleotide monomers connected in a lengthy chain by a covalently bond, is a DNA or RNA strand having at least a certain length. More specifically, it may refer to a polynucleotide fragment encoding the protein.

The polynucleotide encoding the udhA protein of the present disclosure may include a nucleotide sequence encoding the amino acid sequence of SEQ ID NO: 1 or 3. In one example of the present disclosure, the polynucleotide of the present disclosure may have or include a nucleotide sequence of SEQ ID NO: 2 or 4. Additionally, the polynucleotide of the present disclosure may consist of or consist essentially of a nucleotide sequence of SEQ ID NO: 2 or 4. Specifically, the *udhA* gene may be encoded by the polynucleotide represented by the nucleotide sequence of SEQ ID NO: 2 or 4.

The polynucleotide of the present disclosure may undergo various modifications in the coding region within the scope that does not change the amino acid sequence of the soluble pyridine nucleotide transhydrogenase, due to codon degeneracy or in consideration of the codons preferred in an organism in which the soluble pyridine nucleotide transhydrogenase is to be expressed. Specifically, the polynucleotide of the present disclosure may have or include a nucleotide sequence having 70% or more, 75% or more, 76% or more, 85% or more, 90% or more, 95% or more, 96% or more, 97% or more, 98% or more, or 99% or more homology or identity to any one sequence among SEQ ID NO: 2 or 4, or may consist of or consist essentially of a nucleotide sequence having 70% or more, 75% or more, 76% or more, 85% or more, 90% or more, 95% or more, 96% or more, 97% or more, 98% or more, or 99% or more homology or identity to any one sequence among SEQ ID NO: 2 or 4, but is not limited thereto.

In addition, the polynucleotide of the present disclosure may include a probe that may be prepared from a known gene sequence, for example, any polynucleotide sequence which may hybridize with a sequence complementary to all or part of the polynucleotide sequence of the present disclosure under stringent conditions without limitation. The "stringent conditions" refer to conditions which allow the specific hybridization between polynucleotides. Such conditions are disclosed in detail in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, polynucleotides having 70% or more, 75% or more, 76% or more, 85% or more, 90% or more, 95% or more, 96% or more, 97% or more, 98% or more, or 99% or more homology or identity hybridize with each other, while polynucleotides having homology or identity lower than the above homology or identity do not hybridize with each other; or may include ordinary washing conditions of Southern hybridization, i.e., washing once, specifically twice or three times, at a salt concentration and a temperature corresponding to 60°C, 1×SSC, 0.1% SDS, specifically 60°C, 0.1×SSC, 0.1% SDS, and more specifically 68°C, 0.1×SSC, 0.1% SSD.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the present disclosure may also include an isolated nucleic acid fragment complementary to the entire sequence as well as a nucleic sequence substantially similar thereto.

Specifically, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

**The** appropriate stringency for hybridizing polynucleotides depends on the length and degree of complementarity of the polynucleotides, and these variables are well known in the art (*e.g.*, Sambrook *et al., supra*).

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or attenuation of the activity of an endogenous gene, *etc.,* and may be a microorganism including genetic modification to produce a desired polypeptide, protein or product.

Accordingly, in one example of the microorganism of the present disclosure, it may be a recombinant microorganism introduced with an exogenous udhA protein, which has an increased soluble pyridine nucleotide transhydrogenase activity compared to a wild-type microorganism of the genus *Escherichia,* and/or a recombinant microorganism having genetic modification such that the activity of udhA protein is enhanced compared to the endogenous activity thereof.

The microorganism of the present disclosure may have an increased udhA protein activity compared to the endogenous activity thereof. In one example, the microorganism may be a microorganism having an increased L-tryptophan producing ability by introducing an exogenous udhA protein activity. The microorganism may have an enhanced udhA protein activity by introducing an exogenous udhA protein activity, which is not expressed endogenously.

The enhanced udhA protein activity may mean exhibiting an exogenous udhA protein activity, which was not originally possessed by a microorganism, because the udhA protein, which is not expressed endogenously, is introduced.

For example, the microorganism of the present disclosure may have an enhanced soluble pyridine nucleotide transhydrogenase activity within the microorganism, by introducing an exogenous udhA protein having an increased soluble pyridine nucleotide transhydrogenase activity compared to a wild-type microorganism of the genus *Escherichia,* but is not limited thereto.

As used herein, the term "enhancement" of a polypeptide (*e.g.,* proteins specified by the name of each enzyme) means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. **The** "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". **The** "enhancement", "up-regulation", "overexpression", or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide can be confirmed by the increase in the level of activity of the polypeptide, expression level, or the amount of product excreted from the polypeptide.

For the purpose of the present disclosure, the microorganism of the present disclosure has an enhanced soluble pyridine nucleotide transhydrogenase activity within the microorganism, thereby enhancing L-tryptophan producing ability, by introducing an exogenous udhA protein having an increased soluble pyridine nucleotide transhydrogenase activity compared to a wild-type microorganism of the genus *Escherichia,* and the non-modified microorganism not introduced with the exogenous udhA protein, which is the target strain for comparing the increase in the L-tryptophan producing ability or soluble pyridine nucleotide transhydrogenase activity, may be the CA04-4303 strain (US 10995378 B2), which is an L-tryptophan-producing strain, the *E. coli* W3110 strain, or a strain in which one or more genetic modifications are added to the above strains in order to enhance the L-tryptophan production pathway, but is not limited thereto.

The enhancement of the activity of the polypeptide can be applied by various methods well known in the art, and the method is not limited as long as it can enhance the activity of the target polypeptide compared to that of a microorganism before modification. Specifically, genetic engineering and/or protein engineering well known to those skilled in the art, which is a common method of molecular biology, may be used, but the method is not limited thereto (e.g., Sitnicka et al. Functional Analysis of Genes. Advances in Cell Biology. 2010, Vol. 2. 1-16; Sambrook et al. Molecular Cloning 2012*; etc*.).

Specifically, the enhancement of the activity of the polypeptide of the present disclosure may be achieved by:
1) increasing the intracellular copy number of a polynucleotide encoding the polypeptide;
2) replacing the expression regulatory region of a gene encoding the polypeptide on the chromosome with a sequence having a stronger activity;
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same;
9) regulating the cellular localization of the protein(polypeptide); or
10) a combination of two or more selected from above 1 to 9), but is not particularly limited thereto.

### More specifically,

The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto.

Examples of the known strong promoter may include PlysCm1 promoter (US 2023-0134555 A1), CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The 9) method of regulating the cellular localization of the protein(polypeptide) may be achieved by having the protein(polypeptide) target the specific intracellular organelle or specific intracellular space, for example, having the protein(polypeptide) target periplasm or cytoplasm by addition or elimination of leader sequence functioning in targeting of protein(polypeptide), but is not limited thereto.

Such enhancement of the polypeptide activity may mean that the activity or concentration of the corresponding polypeptide is increased relative to the activity or concentration of the polypeptide expressed in a wild-type strain or a microorganism before modification, or that the amount of product produced from the microorganism wherein the polypeptide activity is increased, but is not limited thereto.

In one example, the enhancement of the soluble pyridine nucleotide transhydrogenase activity of the present disclosure may be enhancement by the introduction of an exogenous polynucleotide exhibiting the soluble pyridine nucleotide transhydrogenase activity, but is not limited thereto.

The modification of a part or all of the polynucleotide in the microorganism of the present disclosure may be achieved by (a) homologous recombination using a vector for chromosomal insertion in the microorganism or genome editing using an engineered nuclease (e.g., CRISPR-Cas9), and/or (b) may be induced by light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

The vector of the present disclosure may include a DNA construct containing a nucleotide sequence of a polynucleotide encoding the target polypeptide operably linked to a suitable expression regulatory region (expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL, and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

In addition, as used herein, the term "operably linked" means that the polynucleotide sequence is functionally linked to a promoter sequence that initiates and mediates transcription of the polynucleotide encoding the target polypeptide of the present disclosure.

In another example of the present disclosure, the microorganism of the present disclosure may be a microorganism having an L-tryptophan producing ability.

The microorganism of the present disclosure may be a microorganism having an increased L-tryptophan producing ability.

The microorganism of the present disclosure may be one having an enhanced soluble pyridine nucleotide transhydrogenase activity. Specifically, the microorganism of the present disclosure may be a microorganism, in which an exogenous udhA protein or the *udhA* gene encoding the protein is enhanced; or a microorganism genetically modified to further enhance an exogenous udhA protein or the *udhA* gene encoding the protein, but is not limited thereto. The microorganism, in which an exogenous udhA protein or the *udhA* gene encoding the protein is enhanced, may have an increased soluble pyridine nucleotide transhydrogenase activity compared to a wild-type or a microorganism of the genus *Escherichia* before modification. For example, the microorganism of the present disclosure may be a recombinant microorganism.

The microorganism of the present disclosure may be a microorganism in which an L-tryptophan producing ability is increased or imparted by introducing an exogenous udhA protein or a polynucleotide encoding the protein to a microorganism naturally having an L-tryptophan producing ability or a parent strain having no L-tryptophan producing ability, but is not limited thereto.

For the purpose of the present disclosure, the recombinant microorganism of the present disclosure may be a microorganism having an increased an L-tryptophan producing ability by introducing the exogenous udhA protein or a polynucleotide encoding the protein to a natural wild-type microorganism or a microorganism for producing L-tryptophan, which includes a protein having an endogenous soluble pyridine nucleotide transhydrogenase activity or a polynucleotide encoding the protein, compared to the natural wild-type microorganism or the microorganism for producing L-tryptophan, which includes a protein having an endogenous soluble pyridine nucleotide transhydrogenase activity or a polynucleotide encoding the protein, but is not limited thereto. In one example, the natural wild-type microorganism or the microorganism for producing L-tryptophan, which includes a protein having an endogenous soluble pyridine nucleotide transhydrogenase activity or a polynucleotide encoding the protein, may be the target strain for comparing the increase in the L-tryptophan producing ability or soluble pyridine nucleotide transhydrogenase activity, but is not limited thereto.

In one example, the recombinant strain having an increased production ability may have an increased L-tryptophan producing ability by about 1% or more, specifically about 2.5% or more, about 5% or more, about 7.5% or more, about 10% or more, about 12.5% or more, about 15% or more, about 17.5% or more, about 18% or more, about 20% or more, about 22.5% or more, about 25% or more, about 27.5% or more, about 30% or more, about 32.5% or more, about 35% or more, or about 37% or more (the upper limit is not particularly limited, for example, about 200% or less, about 150% or less, about 100% or less, about 50% or less, about 40% or less, about 30% or less, about 20% or less, or about 15% or less) as compared to the L-tryptophan producing ability of a parent strain before modification or a non-modified microorganism, but is not limited thereto, as long as it has an increased + value compared to the production ability of a parent strain before modification or a non-modified microorganism. In another example, the microorganism having an increased production ability may have an increased L-tryptophan producing ability by about 1.01 times or more, about 1.025 times or more, about 1.05 times or more, about 1.075 times or more, about 1.1 times or more, about 1.125 times or more, about 1.15 times or more, about 1.175 times or more, about 1.18 times or more, about 1.2 times or more, about 1.225 times or more, about 1.25 times or more, about 1.275 times or more, about 1.3 times or more, about 1.325 times or more, about 1.35 times or more, or about 1.37 times or more (the upper limit is not particularly limited, for example, about 10 times or less, about 5 times or less, about 3 times or less, or about 2 times or less) as compared to that of a parent strain before modification or a non-modified microorganism, but is not limited thereto.

The production ability may be evaluated by measuring the production amount of the desired product obtained by culturing in a medium. The evaluation can measure the production amount of the desired product using a suitable method known in the art. For example, HPLC (high performance liquid chromatography), GC (gas chromatography), GC/MS (gas chromatography-mass spectrometry), LC/MS (liquid chromatography-mass spectrometry), GPC (gel permeation chromatography), or a combination thereof may be used, and the production amount of the desired product may be measured using suitable methods known in the art.

As used herein, the term "non-modified microorganism" does not exclude a strain containing a mutation that may occur naturally in a microorganism, and may refer to a wild-type strain or natural-type strain itself, or a strain before the trait is altered due to genetic modification caused by natural or artificial factors. For example, the non-modified microorganism may refer to a strain, in which the soluble pyridine nucleotide transhydrogenase described herein or a polynucleotide encoding the same is not enhanced, or a strain before enhancement thereof. The "non-modified microorganism" may be used interchangeably with "strain before modification", "microorganism before modification", "non-mutant strain", "non-modified strain", "non-mutant microorganism", or "reference microorganism".

In still another example of the present disclosure, the microorganism of the present disclosure is not particularly limited by its type as long as it can produce L-tryptophan, but it may be a microorganism belonging to the genus *Escherichia.* In one example, it may be *Escherichia coli.*

In yet another example of the present disclosure, the recombinant microorganism of the present disclosure may be a microorganism having an enhanced L-tryptophan producing ability by further enhancing the activity of some proteins in the L-tryptophan biosynthesis pathway or by further weakening the activity of some proteins in the L-tryptophan degradation pathway.

In one embodiment, the recombinant microorganism of the present discourse may be a microorganism in which the activity of a gene in a competitive pathway, a regulator in a directional pathway of an L-tryptophan operon, a gene for importing L-tryptophan, or a gene for importing and decomposing L-tryptophan is weakened or inactivated, so as to enhance the L-tryptophan biosynthesis pathway; and/or may be a microorganism in which the activity of an L-tryptophan operon is overexpressed.

In another embodiment, the recombinant microorganism of the present discourse may be a microorganism, in which the activity of trpR, a gene for regulating an enzyme group of tryptophan synthesis that inhibits the expression of L-tryptophan biosynthesis genes (trpEDCBA), or Mtr (i.e., a membrane protein) that imports extracellular L-tryptophan into a cell, may be weakened or removed compared to the endogenous activity thereof, but is not limited thereto.

As used herein, the term "weakening" of the activity of a polypeptide is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the polypeptide activity itself is decreased or removed compared to the activity of the polypeptide originally possessed by a microorganism due to mutation of a polynucleotide encoding the polypeptide; a case where the overall level of intracellular polypeptide activity and/or concentration (expression level) is decreased compared to a natural strain due to the inhibition of expression of a gene of a polynucleotide encoding the polypeptide, or the inhibition of translation into the polypeptide, *etc.;* a case where the polynucleotide is not expressed at all; and/or a case where no polypeptide activity is observed even when the polynucleotide is expressed. As used herein, the term "endogenous activity" refers to the activity of a particular polypeptide originally possessed by a parent strain before transformation, a wild-type or a non-modified microorganism, when a trait is altered through genetic modification caused by natural or artificial factors, and may be used interchangeably with "activity before modification". The expression that the polypeptide activity is "weakened, inactivated, deficient, decreased, down-regulated, reduced or attenuated" compared to its endogenous activity means that the polypeptide activity is decreased compared to the activity of a particular polypeptide originally possessed by a parent strain before transformation or a non-modified microorganism.

The weakening of the polypeptide activity can be performed by any method known in the art, but the method is not limited thereto, and can be achieved by applying various methods well known in the art (e.g., Nakashima N et al., Bacterial cellular engineering by genome editing and gene silencing. Int J Mol Sci. 2014;15(2):2773-2793; Sambrook et al. Molecular Cloning 2012; *etc*.)*.*

Specifically, the weakening of the polypeptide activity of the present disclosure may be achieved by:
1) deleting a part or all of a gene encoding the polypeptide;
2) modifying the expression regulatory region (expression regulatory sequence) such that the expression of a gene encoding the polypeptide is decreased;
3) modifying the amino acid sequence constituting the polypeptide such that the polypeptide activity is removed or weakened (e.g., deletion/substitution/addition of one or more amino acids on the amino acid sequence);
4) modifying the gene sequence encoding the polypeptide such that the polypeptide activity is removed or weakened (e.g., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon, the Shine-Dalgarno (SD) sequence, or 5'-UTR of the gene transcript encoding the polypeptide;
6) introducing an antisense oligonucleotide (e.g., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the polypeptide;
9) regulating the cellular localization of the protein(polypeptide); or
10) a combination of two or more selected from the methods 1) to 9) above, but is not particularly limited thereto.

### For example,

The 1) method of deleting a part or all of the gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating the termination of transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.

The 5) method of modifying the nucleotide sequence encoding the initiation codon or 5'-UTR of the gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (e.g., antisense RNA), which binds complementary to the gene transcript encoding the polypeptide, can be found in the literature (Weintraub, H. et al., Antisense-RNA as a molecular tool for genetic analysis, Reviews - Trends in Genetics, Vol. 1(1) 1986).

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

Further, the 8) reverse transcription engineering (RTE), which adds a promoter, which is to be reversely transcribed, on the 3' terminus of the open reading frame (ORF) of the gene sequence encoding the polypeptide, may be achieved by forming an antisense nucleotide complementary to the gene transcript encoding the polypeptide to weaken the activity.

The 9) method of regulating the cellular localization of the protein(polypeptide) may be achieved by having the protein(polypeptide) target the specific intracellular organelle or specific intracellular space, for example, having the protein(polypeptide) target periplasm or cytoplasm by addition or elimination of leader sequence functioning in targeting of protein(polypeptide), but is not limited thereto.

Such weakening of the polypeptide activity may mean that the activity or concentration of the corresponding polypeptide is decreased relative to the activity or concentration of the polypeptide expressed in a wild-type strain or a microorganism before modification, or that the amount of product produced from the microorganism wherein the polypeptide activity is increased, but is not limited thereto.

Another aspect of the present disclosure provides a method for producing L-tryptophan, including: culturing a microorganism of the genus *Escherichia* for producing L-tryptophan, into which an exogenous udhA protein or a polynucleotide encoding the protein is introduced, in a medium.

The method for producing L-tryptophan of the present disclosure may include culturing a microorganism, in which an exogenous udhA protein or the *udhA* gene encoding the protein is enhanced; or a microorganism genetically modified to further enhance an exogenous udhA protein or the *udhA* gene encoding the protein, in a medium, and the microorganism is as described above.

The exogenous udhA protein may have an increased soluble pyridine nucleotide transhydrogenase activity compared to a wild-type microorganism of the genus *Escherichia.* Additionally, the exogenous udhA protein may not be endogenously expressed in the wild-type microorganism of the genus *Escherichia.*

As used herein, the term "cultivation" means that the microorganism of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the microorganism to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (i.e., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, carbon dioxide, or no gas may be injected to maintain anaerobic or microaerobic conditions, but the gas is not limited thereto.

The temperature during the cultivation of the present disclosure may be in the range from 20°C to 45°C, specifically from 25°C to 40°C, and the cultivation may carried be out for about 10 to 160 hours, but the cultivation is not limited thereto.

The L-tryptophan produced by the cultivation of the present disclosure may be released into the medium or remain in the cells.

The method for producing L-tryptophan of the present disclosure may further include a step of preparing the microorganism of the present disclosure, a step of preparing a medium for culturing the microorganism, or a combination thereof (regardless of the order, in any order), for example, prior to the culturing step.

The method for producing L-tryptophan of the present disclosure may further include a step of recovering L-tryptophan from the cultured medium (medium on which the culture was grown) or the cultured microorganism. The recovering step may be further included after the culturing step.

In the recovering step, the desired L-tryptophan may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* **HPLC** or a combination thereof may be used, and the desired L-tryptophan can be recovered from the medium or the microorganisms using suitable methods known in the art.

Further, the method for producing L-tryptophan of the present disclosure may further include a purification step, which may be performed using an appropriate method known in the art. **In** one example, when the method for producing L-tryptophan of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the exogenous udhA protein, polynucleotide, vector, and microorganism, *etc.,* are as described in other aspects above.

Still another aspect of the present disclosure provides a composition for producing L-tryptophan, including a microorganism of the genus *Escherichia* into which an exogenous udhA protein or a polynucleotide encoding the protein is introduced; a medium on which the microorganism is cultured; or a combination thereof.

The composition of the present disclosure may further include any suitable excipient commonly used in compositions for producing L-tryptophan, and such excipients include, for example, preservatives, wetting agents, dispersing agents, suspending agents, buffers, stabilizers, or isotonic agents, *etc.,* but are not limited thereto.

Yet another aspect of the present disclosure provides a method for preparing a microorganism of the genus *Escherichia* for producing L-tryptophan, including: introducing an exogenous udhA protein or a polynucleotide encoding the same.

The exogenous udhA protein, polynucleotide encoding the protein, and microorganism are as described above.

The preparation method may include a step of modifying the microorganism so that the microorganism has an increased soluble pyridine nucleotide transhydrogenase activity compared to the endogenous activity thereof. This step involves introducing an exogenous udhA protein into the microorganisms of the genus *Escherichia.*

Even another aspect of the present disclosure provides the use of a microorganism of the genus *Escherichia,* into which an exogenous udhA protein or a polynucleotide encoding the protein is introduced, for the production of L-tryptophan.

The exogenous udhA protein, microorganism of the genus *Escherichia, etc.,* are as described in other aspects above.

### [Mode for Carrying Out the Invention]

Hereinafter, the present disclosure will be described in detail by way of Examples. However, these Examples are merely preferred Examples provided for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Screening and Selection of Genes Encoding Soluble Pyridine Nucleotide Transhydrogenase (udhA)

As a result of a PSI-BLAST screen based on the NCBI and KEGG databases with the amino acid sequence of *E. coli*-derived udhA protein as a query sequence, candidate proteins, which are expected to have the activity of catalyzing the reaction of NAD⁺ + NADPH ↔ NADH + NADP⁺, genes encoding these proteins, and microorganisms possessing these genes were determined. Among these, six candidate proteins were selected in consideration of biosafety levels, which are applicable to the production of amino acids, and availability of the produced amino acids, and the results are shown in Table 1 below.

**[Table 1]**

| No. | Strain | Protein Registration No. | Genome Registration No. | Biosafety Level |
|---|---|---|---|---|
| 1 | *Pseudomonas aeruginosa* | WP_003091177.1 | NZ_CP034244.1 | 1 |
| 2 | *Erwinia amylovora* | WP_004154881.1 | NC_013961.1 | 1 |
| 3 | *Alteromonas mediterranea* | AFV86869.1 | CP003917.1 | 1 |
| 4 | *Spongiibacter* sp. IMCC21906 | WP_047011675.1 | NZ_CP011477.1 | 1 |
| 5 | *Amycolatopsis albispora* | AXB47769.1 | CP015163.1 | 1 |
| 6 | *Azotobacter vinelandii* | WP_012700102.1 | NZ_FPKM01000023. 1 | 1 |

### Example 2: Preparation of Plasmids for Gene Insertion

In order to insert the genes into the chromosomes of the *Escherichia,* pSKH (US 8945907 B2) was used to prepare plasmids.

In order to extract the genomic DNA of the wild-type E. *coli* W3110, the Genomic-tip system from Qiagen, Inc. was used. The respective gene fragments were obtained by amplifying the upstream region and the downstream region, where homologous recombination occurs on the chromosomes of the E. coli-derived *udhA* genes, based on the obtained genomic DNA as a template. Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

Additionally, in order to amplify the exogenous *udhA* gene fragments shown in Table 1 above for the replacement of the existing *E*. *coli*-derived *udhA* gene, PCR was performed using the primer pairs shown in Table 2 below based on the *udhA* genes of the *P. aeruginosa, E. amylovora, A. mediterranea, Spongiibacter* sp. IMCC21906, *A. albispora* and *A. vinelandii* strains synthesized using the Gene-Synthesis service by Bionics Co., Ltd. as a template to amplify the *P. aeruginosa-derived udhA* gene (NZ_CP034244.1, SEQ ID NO: 2), E. *amylovora-derived udhA* gene (NC_013961.1, SEQ ID NO: 4), *A. mediterranea-derived udhA* gene (CP003917.1, SEQ ID NO: 6), *Spongiibacter* sp. IMCC21906-derived *udhA* gene (NZ_CP011477.1, SEQ ID NO: 8), *A. albispora-derived udhA* gene (CP015163.1, SEQ ID NO: 10), and *A. vinelandii-derived udhA* gene (NZ_FPKM01000023.1, SEQ ID NO: 12). Solg^{™} Pfu-X DNA polymerase was used as the polymerase, and the PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

The primer sequences used herein are shown in Table 2 below.

**[Table 2]**

| | SEQ ID NO: | Name of Sequence | Sequence (5'->3') |
|---|---|---|---|
| udhA(P.ae)-1 | 13 | udhA(P.ae)-1_F | CAGGAATTCGATCTGAATTTCACGCGCAA CGG |
| | 14 | udhA(P.ae)-1_R | GTTGTAGACAGCCATGGTAGGGCTTACC TGTTCTTA |
| udhA(P.ae)-2 | 15 | udhA(P.ae)-2_F | CAGGTAAGCCCTACCATGGCTGTCTACA ACTACGAC |
| | 16 | udhA(P.ae)-2_R | CATTTCGATAAAGTTTCAAAAAAGCCGGT TGAGGC |
| udhA(P.ae)-3 | 17 | udhA(P.ae)-3_F | AACCGGCTTTTTTGAAACTTTATCGAAAT GGCCATCC |
| | 18 | udhA(P.ae)-3_R | GCTAGGTCGACTAGCGTGATGGAATGCT GCTGGTGGATCA |
| udhA(E.am)-1 | 13 | udhA(E.am)-1_F | CAGGAATTCGATCTGAATTTCACGCGCAA CGG |
| | 19 | udhA(E.am)-1_R | ATAAGACTTTTGCATGGTAGGGCTTACCT GTTCTTA |
| udhA(E.am)-2 | 20 | udhA(E.am)-2_F | CAGGTAAGCCCTACCATGCAAAAGTCTTA TGATTACGACG |
| | 21 | udhA(E.am)-2_R | CATTTCGATAAAGTTCTAAAATAGCCGGT TCAGGCC |
| udhA(E.am)-3 | 22 | udhA(E.am)-3_F | AACCGGCTATTTTAGAACTTTATCGAAAT GGCCATCC |
| | 18 | udhA(E.am)-3_R | GCTAGGTCGACTAGCGTGATGGAATGCT GCTGGTGGATCA |
| udhA(A.me)-1 | 13 | udhA(A.me)-1_F | CAGGAATTCGATCTGAATTTCACGCGCAA CGG |
| | 23 | udhA(A.me)-1_R | CTTGGCAAGTTGCATGGTAGGGCTTACC TGTTCTTA |
| udhA(A.me)-2 | 24 | udhA(A.me)-2_F | CAGGTAAGCCCTACCATGCAACTTGCCA AGGCCGG |
| | 25 | udhA(A.me)-2_R | CATTTCGATAAAGTTTTAGAAAATCCTGTT CAAGCCGTTT |
| udhA(A.me)-3 | 26 | udhA(A.me)-3_F | AACAGGATTTTCTAAAACTTTATCGAAAT GGCCATCC |
| | 18 | udhA(A.me)-3_R | GCTAGGTCGACTAGCGTGATGGAATGCT GCTGGTGGATCA |
| udhA(S.po)-1 | 13 | udhA(S.po)-1_F | CAGGAATTCGATCTGAATTTCACGCGCAA CGG |
| | 27 | udhA(S.po)-1_R | GTAATCATATGCCATGGTAGGGCTTACCT GTTCTTA |
| udhA(S.po)-2 | 28 | udhA(S.po)-2_F | CAGGTAAGCCCTACCATGGCATATGATTA CGACGTTGTG |
| | 29 | udhA(S.po)-2_R | CATTTCGATAAAGTTTTATAACCGATTTAA GCCATTAAGC |
| udhA(S.po)-3 | 30 | udhA(S.po)-3_F | TTAAATCGGTTATAAAACTTTATCGAAATG GCCATCC |
| | 18 | udhA(S.po)-3_R | GCTAGGTCGACTAGCGTGATGGAATGCT GCTGGTGGATCA |
| udhA(A.al)-1 | 13 | udhA(A.al)-1_F | CAGGAATTCGATCTGAATTTCACGCGCAA CGG |
| | 31 | udhA(A.al)-1_R | GTCATAGGCGCTCATGGTAGGGCTTACC TGTTCTTA |
| udhA(A.al)-2 | 32 | udhA(A.al)-2_F | CAGGTAAGCCCTACCATGAGCGCCTATG ACTACGAC |
| | 33 | udhA(A.al)-2_R | CATTTCGATAAAGTTTCAGGTTGTGGTGA AGCGGG |
| udhA(A.al)-3 | 34 | udhA(A.al)-3_F | TTCACCACAACCTGAAACTTTATCGAAAT GGCCATCC |
| | 18 | udhA(A.al)-3_R | GCTAGGTCGACTAGCGTGATGGAATGCT GCTGGTGGATCA |
| udhA(A.vi)-1 | 13 | udhA(A.vi)-1_F | CAGGAATTCGATCTGAATTTCACGCGCAA CGG |
| | 35 | udhA(A.vi)-1_R | GTTATATACAGCCATGGTAGGGCTTACCT GTTCTTA |
| udhA(A.vi)-2 | 36 | udhA(A.vi)-2_F | CAGGTAAGCCCTACCATGGCTGTATATAA CTACGATGTGG |
| | 37 | udhA(A.vi)-2_R | CATTTCGATAAAGTTTCAAAAAAGCCGAT TGAGACCG |
| udhA(A.vi)-3 | 38 | udhA(A.vi)-3_F | AATCGGCTTTTTTGAAACTTTATCGAAAT GGCCATCC |
| | 18 | udhA(A.vi)-3_R | GCTAGGTCGACTAGCGTGATGGAATGCT GCTGGTGGATCA |

The thus-obtained amplified upstream gene fragments and the downstream gene fragments of the *E. coli*-derived *udhA* gene, the exogenous *udhA* gene fragments, and the vector pSKH for chromosomal transformation cleaved by EcoRV restriction enzyme were cloned using the Gibson assembly method (DG Gibson et al., NATURE METHODS, VOL. 6 NO. 5, May 2009, NEBuilder HiFi DNA Assembly Master Mix) to obtain recombinant plasmids. The cloning was performed by mixing the Gibson assembly reagent and each of the gene fragments in a calculated number of moles followed by incubating at 50°C for 1 hour. The resulting recombinant plasmids were named pSKH-udhA(P.ae), pSKH-udhA(E.am), pSKH-udhA(A.me), pSKH-udhA(S.po), pSKH-udhA(A.al), and pSKH-udhA(A.vi), respectively, according to the species of the replaced *udhA* gene.

### Example 3: Preparation of Microorganisms of the Genus Escherichia Introduced with Exogenous udhA Genes

The pSKH-udhA(P.ae), pSKH-udhA(E.am), pSKH-udhA(A.me), pSKH-udhA(S.po), pSKH-udhA(A.al), and pSKH-udhA(A.vi) plasmids prepared in Example 2 were transformed into CA04-4303 (US 10995378 B2), which is an L-tryptophan-producing strain, by electroporation (Appl. Microbiol. Biotechnol. (1999) 52:541-545) and then subjected to a secondary crossover to obtain strains, in which the *E. coli*-derived *udhA* gene was replaced with the exogenous *udhA* genes on the chromosomes. The corresponding genetic manipulation was confirmed through genome sequencing and a PCR method using a primer pair of SEQ ID NOS: 39 and 40, which can respectively amplify the external region of the upstream region and downstream region of the homologous recombination where the corresponding genes were inserted. The resulting strains, in which the *E. coli*-derived *udhA* gene was replaced with the exogenous *udhA* genes, were named CA04-4303::udhA(P.ae), CA04-4303::udhA(E.am), CA04-4303::udhA(A.me), CA04-4303::udhA(S.po), CA04-4303::udhA(A.al), and CA04-4303::udhA(A.vi), respectively.

The primer sequences used herein are shown in Table 3 below.

**[Table 3]**

| SEQ ID NO: | Name of Sequence | Sequence (5'->3') |
|---|---|---|
| 39 | udhA-confirm F | CAGTAAACGCACGCGGCA |
| 40 | udhA-confirm R | GTGATGTTGCTGGAGCGG |

### Example 4: Confirmation of L-Tryptophan Production Ability of Microorganisms of the Genus Escherichia Introduced with Exogenous udhA Genes

In order to confirm the L-tryptophan producing ability of the CA04-4303::udhA(P.ae), CA04-4303::udhA(E.am), CA04-4303::udhA(A.me), CA04-4303::udhA(S.po), CA04-4303::udhA(A.al), and CA04-4303::udhA(A.vi) strains introduced with the exogenous *udhA* genes prepared in Example 3, and the parent strain CA04-4303 as a control, the strains were cultured in the following manner.

Specifically, one platinum loop of each strain cultured overnight in a LB solid medium in a 37°C incubator was inoculated into 25 ml of a titer medium shown in Table 4 below, and then cultured in an incubator at 37°C at a rate of 200 rpm for 48 hours. After completion of the culture, the production amount of L-tryptophan was measured by HPLC, and the results are shown in Table 5 below.

**[Table 4]**

| **Composition** | **Concentration (per liter)** |
|---|---|
| Glucose | 60 g |
| K₂HPO₄ | 1 g |
| (NH₄)₂SO₄ | 10 g |
| NaCl | 1 g |
| MgSO₄.7H₂O | 1 g |
| Sodium citrate | 5 g |
| Yeast extract | 2 g |
| Calcium carbonate | 40 g |
| Phenylalanine | 0.15 g |
| Tyrosine | 0.1 g |
| pH | 6.8 |

**[Table 5]**

| Name of Strain | OD562 | Production Amount of Tryptophan (g/L) | Tryptophan Yield (*100 g/g, %) |
|---|---|---|---|
| CA04-4303 | 43.3 | 1.37 | 1.96 |
| CA04-4303::udhA(P.ae) | 41.7 | 1.87 | 2.68 |
| CA04-4303::udhA(E.am) | 42.9 | 1.62 | 2.31 |
| CA04-4303:: udhA(A. me) | 43.4 | 1.36 | 1.95 |
| CA04-4303::udhA(S.po) | 43.7 | 1.34 | 1.92 |
| CA04-4303::udhA(A.al) | 43 | 1.29 | 1.85 |
| CA04-4303::udhA(A.vi) | 43.1 | 1.39 | 1.99 |

As shown in Table 5 above, among the six production strains, into which the exogenous *udhA* genes were introduced, the CA04-4303::udhA(P.ae) strain, into which the *P. aeruginosa-derived udhA* gene was introduced, finally produced 1.87 g/L of L-tryptophan during the flask culture, showing an increased fermentation yield by about 37% compared to the control CA04-4303 strain. Additionally, the CA04-4303::udhA(E.am) strain, into which the *E. amylovora-derived udhA* gene was introduced, finally produced 1.62 g/L of L-tryptophan during the flask culture, showing an increased fermentation yield by about 18% compared to the control CA04-4303.

Based on the results above, it was confirmed that among the six exogenous *udhA* genes, the *udhA* genes derived from *P. aeruginosa* and *E. amylovora* specifically increased the L-tryptophan producing ability in *Escherichia coli.*

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A microorganism of the genus *Escherichia,* into which an exogenous udhA protein or a polynucleotide encoding the protein is introduced.

2. The microorganism of claim 1, wherein the protein comprises an amino acid sequence of SEQ ID NO: 1 or 3, or an amino acid sequence having 90% or more identity thereto.

3. The microorganism of claim 1, wherein the protein is derived from any one or more microorganisms selected from the group consisting of *Pseudomonas aeruginosa* and *Erwinia amylovora.*

4. The microorganism of claim 1, wherein the microorganism of the genus *Escherichia* has an increased L-tryptophan producing ability compared to a microorganism of the genus *Escherichia* before modification.

5. The microorganism of claim 1, wherein the microorganism of the genus *Escherichia* is *Escherichia coli.*

6. A method for producing L-tryptophan, comprising: culturing a microorganism of the genus *Escherichia,* into which an exogenous udhA protein or a polynucleotide encoding the protein is introduced, in a medium.

7. The method of claim 6, wherein the protein is derived from any one or more microorganisms selected from the group consisting of *Pseudomonas aeruginosa* and *Erwinia amylovora.*

8. A composition for producing L-tryptophan, comprising a microorganism of the genus *Escherichia,* into which an exogenous udhA protein or a polynucleotide encoding the protein is introduced; a medium on which the microorganism is cultured; or a combination thereof.

9. The composition of claim 8, wherein the protein is derived from any one or more microorganisms selected from the group consisting of *Pseudomonas aeruginosa* and *Erwinia amylovora.*
